# EUROPEAN PATENT APPLICATION

(11) **EP 1 126 023 A1**
(43) Date of publication of application: **22.08.2001**
(21) Application number: 99969943.2
(22) Date of filing: 30.09.1999
(51) Int. Cl.: C12N 5/06, C12N 15/00

(54) **METHOD FOR INDUCING CELLULAR IMMUNITY AND CELLS WITH INDUCED CELLULAR IMMUNITY**

(30) Priority: 02.10.1998 JP 28109798; 09.02.1999 JP 3131699; 12.07.1999 JP 19693899
(71) Applicant: MITSUBISHI CHEMICAL CORPORATION, Chiyoda-ku, Tokyo 100-0005 (JP)
(72) Inventor: SHIKU, Hiroshi, Tsu-shi, Mie 514-0061 (JP); SUNAMOTO, Junzo, Kusatsu-shi, Shiga 525-0045 (JP)
(74) Representative: Sternagel, Fleischer, Godemeyer & Partner Patentanwälte
(86) International application number: JP9905378
(87) International publication number: WO0020563

(57) **Abstract**

A cell with cellular immunity induced by reacting *in vitro* a complex comprising a hydrophobized polysaccharide, such as a polysaccharide modified with an alkyl group or a sterol residue, and an antigen with an antigen-presenting cell such as a dendritic cell. The antigen-presenting cell has cellular immunity induced more efficiently than conventional vaccines, and is useful for a cell therapy.

## Description

### Technical Field

The present invention relates to a method for inducing cellular immunity and cells having induced cellular immunity. More specifically, the invention relates to reaction *in vitro* a complex comprising a hydrophobized polysaccharide and an antigen with antigen-presenting cells, which achieves more effective activation and induction of cellular immunity, particularly cytotoxic T cells (hereinafter abbreviated occasionally as "CTL") that are specific for an antigen, than conventional vaccines.

### Background Art

CTLs are known to specifically recognize cancer cells or virus-infected cells through their T cell receptors and the like to destroy or damage the cells, and accordingly, have important roles as a bio-defense mechanism in *vivo* against cancers or viruses. In addition, it has been recently revealed that T cell receptors of CTL do not directly recognize the specific antigen expressed on the surface of cancer cells or virus-infected cells, but they recognize complexes including an MHC class I antigen, which is expressed on the surface of antigen-presenting cells such as macrophages and dendritic cells, or cancer cells or virus-infected cells, *per se,* together with an oligopeptide derived from the specific antigen bound to the MHC class I antigen ("CTL epitope" of the specific antigen).

It is considered that the complex of an MHC class I antigen/an oligopeptide derived from a specific antigen is formed by a processing as explained below. Antigenic proteins are synthesized in cytoplasm, and then some of the proteins are degraded into oligopeptides by intracellular protease complexes ("proteasome"). Some of the oligopeptides (consisting of 9 to 10 amino acid residues) are then transported from cytoplasm into the endoplasmic reticulum membrane by transporter proteins (TAP: transporter in antigen processing) that are present in the endoplasmic reticulum membrane, and oligopeptides having high affinity for the MHC class I antigen are preferentially bind to the MHC class I antigen and presented on the surfaces of the cells.

Accordingly, in order to artificially activate the CTL for the purpose of therapeutic or preventive treatment of cancers, viral diseases, or autoimmune diseases by eliminating cancer cells, virus-infected cells, or autoantigen-reactive lymphocytes, it is necessary to perform immunization of a living body with cancer cells or virus-infected cells, *per se,* which express specific antigens, or alternatively, to introduce the specific antigens or the oligopeptides derived therefrom into the processing pathway for the antigen-presenting cells so that a complex with the MHC class I antigen can be expressed.

In order to develop such "CTL-activating vaccines", some attempts have been made, which include 1) a method comprising the step of introducing a gene encoding a specific antigenic protein by means of a viral vector and the like; 2) a method comprising the step of introducing a specific antigenic protein, having an appropriate molecular weight and containing several CTL epitopes, into cytoplasm by means of a certain technique; and 3) a method comprising the step of binding an oligopeptide consisting *of 9* to 10 amino acid residues derived from a specific antigen as a potential CTL epitope directly to an MHC class I antigen of an antigen-presenting cell.

Among them, method 1) is a so-called gene therapy, whose efficacy and safety have not yet been established generally. As for the approach according to the method 3), efficacy has been verified in animal studies and the like; however, practical problems may arise when an application to human is developed. Since MHC class I antigens of respective patients widely vary to each other, and it should be necessary to cover diversity of CTL epitopes resulting from the variety of antigens. In other words, it is necessary to elucidate amino acid sequential motifs of oligopeptides having high affinity for each kind of the MHC class I antigens, and to achieve customized manufacture of oligopeptides as medicaments that correspond to each of the MHC class I antigens. Therefore, the development of medicaments based on this class seems to be extremely difficult.

As for the approach according to method 2), some specific examples are known. Therefore, the method is considered as most promising means to develop CTL activating vaccines which is satisfactory from viewpoints of efficacy and safety and is applicable to variety of patients. Practically, when a specific antigenic protein, *per se* as a polypeptide, is administered to a living body to activate specific CTLs, the protein is usually administered as a mixture with an adjuvant. For example, ISCOM (Takahashi et al., Nature, 344, 873-875, 1990), QS-21 (Newman et al., J. Immunol., 148, 2357-2362, 1992), mannan-coated liposomes (WO92/4887), AF (Raychaudhuri et al., Proc. Natl. Acad. Sci. USA, 89, 8308-8312, 1992) and the like have been known.

It is known that polysaccharide/cholesterol derivatives can be used as polysaccharide coating agents for liposomes [Japanese Patent Unexamined Publication (KOKAI) No.(Sho)61-69801/1986], or coating agents for lipid emulsions [Japanese Patent Unexamined Publication (KOKAI) No.(Sho)63-319046/1988]. As explained above, it is also known that tumor cell antigen or virus antigen/liposome complexes coated with polysaccharides containing mannose, i.e., those obtained by a coating process using a polysaccharide/cholesterol derivative after a liposome formation or a lipid emulsification using an (antigenic) protein, have inducing activity of CTL (see, WO92/4887).

It is further known that the complexes consisting essentially of a polysaccharide/cholesterol derivative and an (antigenic) protein is a potentially useful vaccine (see, WO98/9650).

### Disclosure of the Invention

The inventors of the present invention conducted various studies on methods for inducing cellular immunity more efficiently. As a result, they found that when complexes which comprise antigenic proteins and hydrophobized polysaccharides composed of naturally occurring polysaccharides modified with alkyl groups or cholesterol groups are reacted with antigen-presenting cells *in vitro,* cellular immunity is efficiently activated and thereby bio-defense reactions are induced. The present invention was achieved on the basis of these findings.

The present invention thus provides a cell with induced cellular immunity wherein said cellular immunity is induced by reacting *in vitro* a complex comprising a hydrophobized polysaccharide and an antigen with an antigen-presenting cell.

According to preferred embodiments of the present invention, there are provided the aforementioned cell wherein the antigen-presenting cell is a dendritic cell; the aforementioned cell characterized in that the hydrophobized polysaccharide is a polysaccharide modified with an alkyl group or a sterol residue; the aforementioned cell wherein the hydrophobized polysaccharide is a polysaccharide
characterized to contain a saccharide unit, at a ratio of 0.5 to 5 in average per 100 saccharide units that constitute the polysaccharide, whose primary hydroxyl group is a group represented by the formula:

-O-(CH₂)ₘCONH(CH₂)ₙNH-CO-O-R

wherein R represents an alkyl group or a sterol residue; m represents 0 or 1; and n represents an arbitrary positive integer; the aforementioned cell characterized in that the sterol residue is cholesterol residue; the aforementioned cell characterized in that the polysaccharide is pullulan or mannan; the aforementioned cell characterized in that the antigen is a protein which is presented as an oligopeptide by an MHC class I antigen and induces a cytotoxic T-cell; the aforementioned cell wherein the antigen is a tumor cell antigen, a viral antigen, or an autoantigen-reactive T-cell receptor; the aforementioned cell characterized in that the antigen is ErbB-2 protein; and the aforementioned cell which is used as a medicament for parenteral administration.

As another aspect of the present invention, there is provided a method for inducing cellular immunity characterized in that the method comprises the step of reacting *in vitro* a complex comprising a hydrophobized polysaccharide and an antigen with an antigen-presenting cell. According to preferred embodiments of this invention, there are provided the aforementioned method characterized in that an amount of the complex comprising a hydrophobized polysaccharide and an antigen is sufficient to induce cellular immunity; a method which comprises the steps of isolating an antigen-presenting cell from a living body, reacting a complex comprising a hydrophobized polysaccharide and an antigen with the antigen-presenting cell, and returning the resulting cell to the living body; and the aforementioned method which comprises the step of returning to the living body by parenteral administration.

### Brief Explanation of Drawings

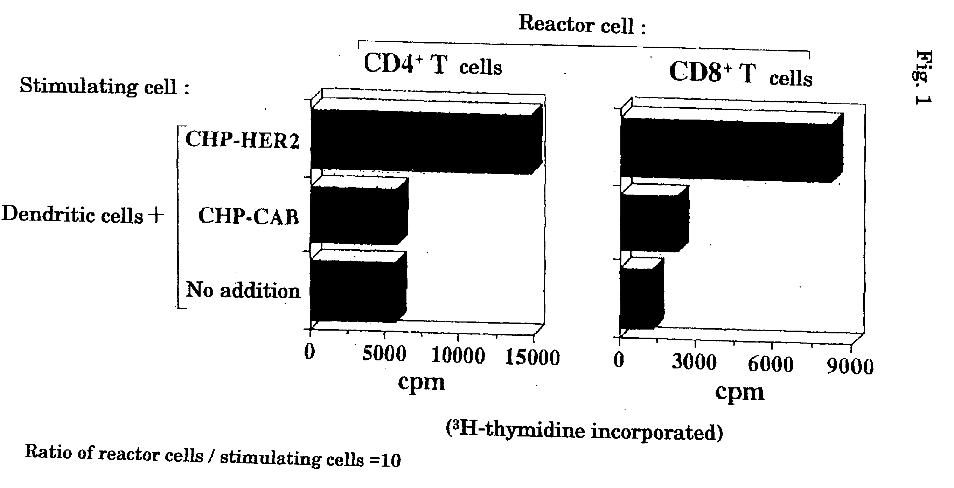

Fig. 1 depicts the growth of CD4-positive T cells and CD8-positive T cells isolated from mice. The vertical axis represents the mouse dendritic cells treated with CHP-HER2 and CHP-CAB and the non-treated dendritic cells, and the horizontal axis represents the amount of ³H thymidine incorporated.

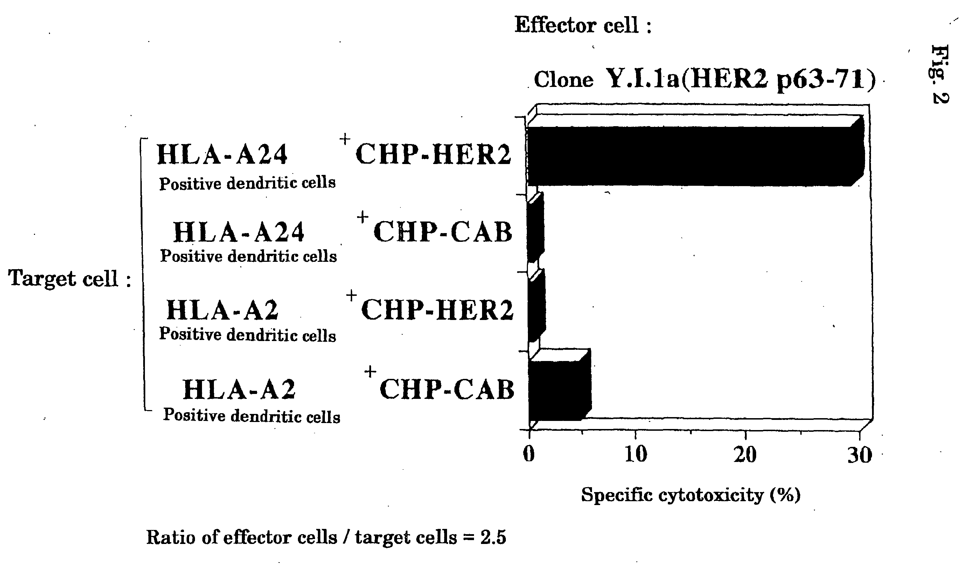

Fig. 2 depicts the cytotoxicity of CTL clones against the dendritic cells isolated from healthy humans and then treated with CHP-HER2 or CHP-CAB. The upper two bars in the vertical axes represent the results obtained by the dendritic cells derived from healthy humans having HLA-A2402, and the bottom two bars represent the results obtained by the dendritic cells derived from healthy humans having HLA-A0201, both after the treatment with CHP-HER2 or CHP-CAB. The horizontal axis represents the cytotoxicity by the CTL clones in the Cr⁵¹ releasing test.

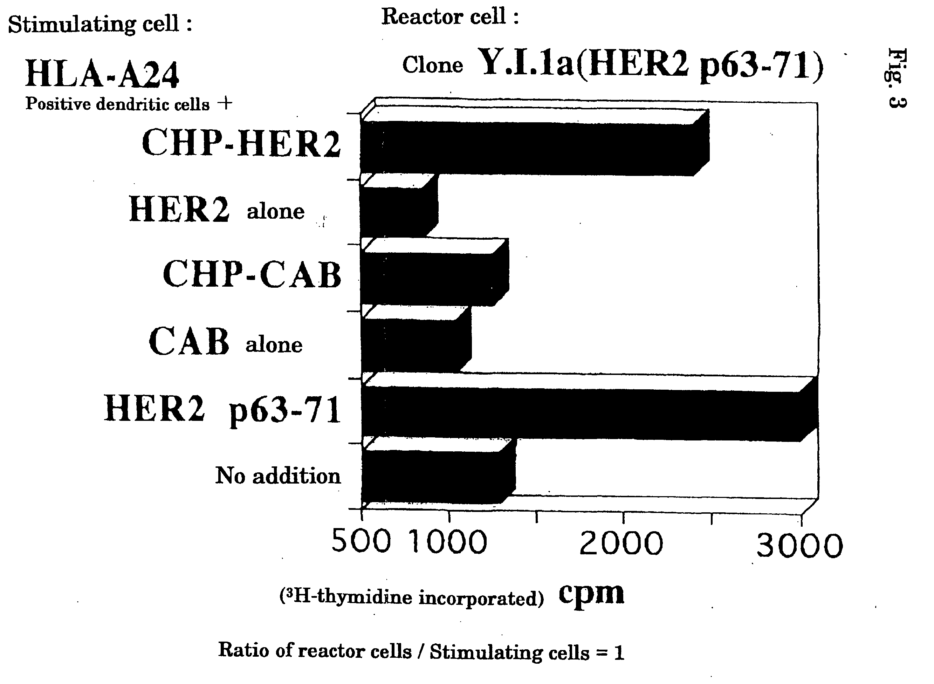

Fig. 3 depicts the growth of CTL clones when the dendritic cells isolated from healthy humans were treated with CHP-HER2, HER2, CHP-CAB, CAB, or HER2p63-71. The respective bars in the vertical axes represent the dendritic cells treated with CHP-HER2, HER2, CHP-CAB, CAB, and HER2p63-71 (positive controls), and the non-treated dendritic cells. The horizontal axis represents the growth ability of the CTL clones (HER2p63-71)

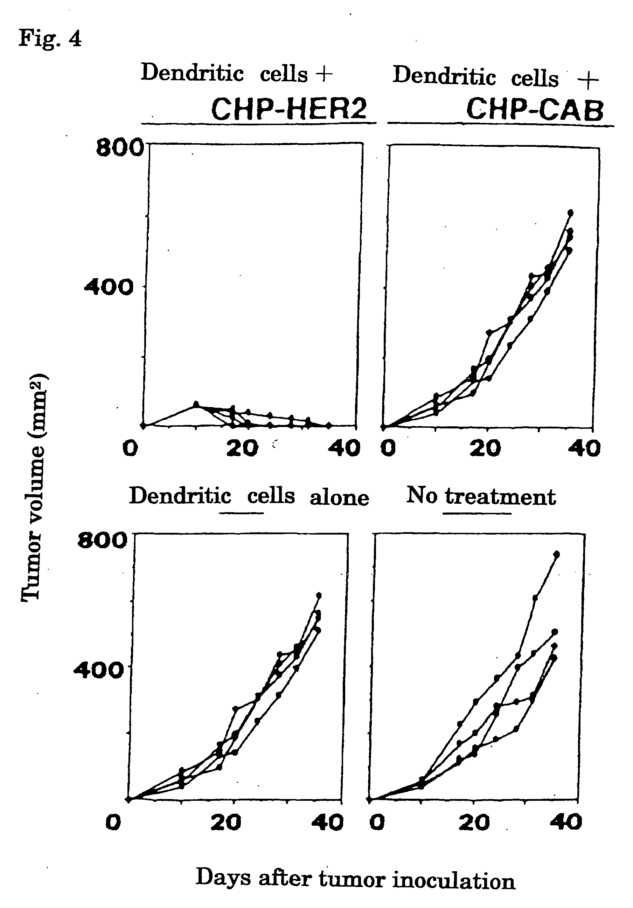

Fig. 4 depicts the growth curve of tumor cells on mouse individuals when the mice inoculated with mouse tumor cells CMS7HE expressing human ErbB-2 were immunized with dendritic cells which were treated with a complex of cholesterol-modified pullulan and human ErbB-2 protein as an oncogene product or the like. The vertical axis represents the tumor size (mm²) obtained by calculation after measurement of the major axis and the minor axis, and the horizontal axis represents the number of days after tumor inoculation. In the figure, DC (CHP-HER2), DC (CHP-CAB) (control), and DC represent the data obtained from mice immunized with the dendritic cells treated with CHP-ErbB2, the cells treated with CHP-CAB, and the cells without treatment, respectively. "No treatment" represents data from the non-immunized mice.

### Best Mode for Carrying Out the Invention

The present invention will be explained in detail below. According to the present invention, the term "adjuvant" means a substance which is used together with an antigen to modify an immune response against the antigen. Generally, adjuvants are used enhancing antibody production and cellular immunity, and optionally encompass those used for negatively changing the immune response.

### (1) Antigen

The antigens used in the present invention are substances that induce immunity such as polypeptides, polypeptide complexes, glycoproteins, nucleic acids and the like. The antigens may be a part of the pathological target, *per* se, or those produced by focal tissues such as neoplastic tissues and virus-infected cells. According to the present invention, antigenic proteins, in particular, those presented as oligopeptides by MHC class I antigens and inducing CTLs may be used.

The origin and purity of the antigenic proteins are not particularly limited so far that they contain such antigenic determinants. Desirably, those produced by genetic recombinant techniques are preferred. Molecular weight of the antigenic proteins is not particularly limited so far that they contain the antigenic determinants, and may generally be from 500 to 100,000, preferably from 2,000 to 100,000. As the peptide fragment, those having 30 or more amino acids are preferably used. Specific examples include oncogene products, ErbB-2 protein (abbreviated occasionally as HER2), ras p21 protein, tumor suppressor gene product p53 protein, virus derived protein, and T cell receptors such as a whole protein of an autoantigen-reactive (autoimmune disease-inducing) T cell receptor and a protein containing a partial sequence thereof.

### (2) Hydrophobized polysaccharide

As a method for preparing the hydrophobized polysaccharides and a method for preparing the microparticles comprising hydrophobized polysaccharide aggregates, the method of Akiyoshi et al. may be used (Akiyoshi et al., Macromolecules, 26, 3062-3068 (1993); Akiyoshi et al., J. Proc. Japan. Acad., 71, 71B, 15 (1995); Japanese Patent Unexamined Publication (KOKAI) Nos.(Sho)61-69801/1986, (Hei)3-292301/1991, and (Hei)7-97333/1995).

Polysaccharides constituting the hydrophobized polysaccharide are polymers composed of monosaccharide residues linked each other by glycoside bonds. The saccharide components may be derived from monosaccharides such as glucose, mannose, galactose and fucose; disaccharides; oligosaccharides and the like. The saccharide units may be linked with 1,2-, 1,3-, 1,4- or 1,6-glycoside bond, and the bond may be either in α - or β -linkage. The chain may be linear or branched. The polysaccharides containing glucose as a saccharide component are preferred, and more specifically, naturally occurring or synthesized pullulan, dextran, amylose, amylopectin, and mannan, and preferably, mannan and pullulan may be used.

According to the present invention, the hydrophobized polysaccharides are prepared by introducing hydrophobic groups into the aforementioned polysaccharides. The hydrophobized polysaccharides may be those connected by means of linkers such as described in Japanese Patent Unexamined Publication (KOKAI) No.(Hei)7-97333/1995.

The hydrophobic groups include, in general, alkyl groups, sterol groups and the like, and sterol residues are preferred. As for the hydrophobic ratio, the polysaccharides have, for example, from 0.5 to 5 alkyl groups or sterol residues in average, preferably from 1 to 5 in average, per 100 monosaccharides. In addition, those having 5% by weight or less of the hydrophobic groups are desired. However, the groups are not particularly limited to the above examples, and those achieving high encapsulating ratio can be used depending on the molecular weight and isoelectric point of an antigen to be encapsulated. The sterol residues include, for example, cholesterol, stigmasterol, β -sitosterol, lanosterol, ergosterol residues and the like, and cholesterol residue can be preferably used. The alkyl groups include, for example, lauryl group, stearyl group, palmityl group and the like, and particularly those having 20 or less of carbon atoms, preferably 10 to 18 carbon atoms. The alkyl groups may be linear or branched.

The hydrophobized polysaccharide according to the present invention is formed by binding the primary hydroxyl group of the polysaccharide to the aforementioned hydrophobic group by means of an optional connecting group. Examples of the connecting group include, for example, those represented by the following formula. The hydrophobized polysaccharide may have two or more sterol residues or alkyl groups binding to one hydroxyl group of the polysaccharide:

-O-(CH₂)ₘCONH(CH₂)ₙNH-CO-O-

wherein m represents 0 or 1; and n represents an arbitrary positive integer, preferably an integer of from 1 to 8.

### (3) Method for encapsulating antigens by using the hydrophobized polysaccharide

The complexes comprising the hydrophobized polysaccharide and the antigen can be isolated and purified, for example, by mixing microparticles of aggregated hydrophobized polysaccharide and antigens at room temperature, and then treating the mixture by gel chromatography (Nishikawa, Macromolecules, 27, 7654-7659 (1994)).

The complexes having the particle diameter of from about 5 to 50 nm can be prepared by mixing the hydrophobized polysaccharide and the antigen at a ratio by weight of 0.1 to 100 : 1 as described in, for example, the following two articles: J. Am. Chem. Soc., 118, 6110-6115 (1996); and Macromolecules, 27, 7654-7659 (1994).

The complexes of the hydrophobized polysaccharide and the antigen thus obtained, *per se,* can be used in the present invention, or they may be subjected to further treatments such as sterilization by standard methods, if necessary.

### (4) Antigen-presenting cells

The term "antigen-presenting cell" means a cell enhancing the activity of lymphocytes by transmitting information to lymphocytes about a foreign substance that invaded in a living body, which triggers the activation of immune system. Examples include dendritic cells, macrophages (phagocytes), B cells, Kupffer cells in the liver, thymic epithelial cells and the like. These cells express major histocompatibility antibodies (MHC) class II as well as major histocompatibility antibodies (MHC) class I. The antigen-presenting cells include, for example, those derived from vertebrate animals, preferably mammals, more preferably humans.

As for a method for obtaining dendritic cells, for example, the cells can be obtained by isolating bone marrow cells from animals (e.g., mice), treating the cells with an antibody or the like, and then culturing the resulting marrow stem cells in the presence of the granulocyte macrophage colony-stimulating factor (GM-CSF) or the like for a few days. However, the method is not limited to the above example. Alternatively, for example, the cells can be obtained by isolating and culturing human peripheral blood mononuclear cells by the Ficoll-Conray method or the like, and culturing the adherent cells in the presence of GM-CSF and interleukin (IL-4) or the like for a few days.

### (5) Method for reacting the complex with the antigen-presenting cells (dendritic cells)

The reaction may be carried out by adding a suspension of the complex comprising the hydrophobized polysaccharide and the antigen obtained in the above (3) to the dendritic cells obtained in the above (4), preferably at the protein concentration of 100 *µ* g/ml, and culturing the resulting mixture at the culturing temperature of from about 1 to 50°C, preferably around 37°C, for from about 1 to 48 hours.

### (6) Induction of cellular immunity

Cellular immunity is induced by the reaction of the aforementioned complex with the dendritic cells. In the reaction, the aforementioned complex may be reacted in an amount sufficient to induce cellular immunity. A determination whether or not cellular immunity is induced can be conducted by co-culturing the resulting dendritic cells and T cells such as CD4-positive T cells, CD8-positive T cells, and CTL clones prepared so as to recognize peptides (e.g., HER2p63-71, a CTL clone prepared so as to recognize the peptide having the 63-71 amino acid sequence of HER2 which is known to act specifically on MHC class I), and then observing cytotoxic ability of the T cells against the dendritic cells, and their ability of stimulation or proliferation by using a radioisotopes or the like.

The mechanism of inducing cellular immunity by the aforementioned complex has not been fully revealed; however, it is presumed that the induction may proceed through a processing pathway of the conventional MHC class I antigen/an oligopeptide complex described in paragraph [0003]. This mechanism can be presumed by results of simultaneous treatments with agents which selectively inhibit respective steps in the pathway as described below.

Dendritic cells are pretreated for from 1 to 3 hours with an agent such as 1) Cytochalasin D which inhibits active phagoytosis (e.g., 30 *µ* g/ml), 2) Lactacystin as a proteosome inhibitor (e.g., 100 *µ* M), 3) Cycloheximide which inhibits new biosynthesis of MHC class I antigens (e.g., 10 *µ* g/ml), and 4) Brefeldin A which inhibits the transition of a protein from endoplasmic reticulum (ER) to Goldi body (e.g., 5 *µ* g/ml), and additionally the cells are treated beforehand similarly in the reaction with the complex. As the cytotoxic ability of the aforementioned T cells is reduced compared to untreated control group, it can be presumed that the mechanism involves the pathway. In addition, a comparative experiment can be conducted at the same time to observe ineffectiveness of the aforementioned inhibitors when the dendritic cells are reacted directly with an oligopeptide as an antigenic epitope instead of the complex.

Moreover, the cytotoxic ability of the T cells is not inhibited by treatment with 5) Amiloride which inhibits passive macropinocytosis (e.g., 0.2 mM), or 6) Chloroquine (e.g., 30 *µ* M) which inhibits binding of an oligopeptide, prepared by degradation of a protein in endosome, to MHC class II antigens. Accordingly, it can be presumed that the mechanism of inducing cellular immunity by the aforementioned complex does not involve a pathway other than the conventional pathway based on slow release of a protein by the aforementioned composition.

Cellular immunity can be induced *in vivo* by returning the cells obtained in the above (5) to a living body.

For example, dendritic cells with induced cellular immunity can be returned to a living body by means of injections or the like (e.g., in blood or bone marrow) in a state of maintained activation, for example, in a solution containing a conventionally used buffer or blood components and the like. For example, cells can be returned to a living body by the methods described in the following three articles: NATURE MEDICINE, 4(3), 328-332 (1998); Proc. Natl. Acad. Sci. USA, 92, 8078-8082 (1995); and Canser Res., 56, 2479-2483 (1996).

The cells with induced cellular immunity according to the present invention can be parenterally administered, in general, together with a solution containing a buffer (e.g., HBSS, PBS or the like), physiological saline, blood ingredients or the like. Examples of methods for administration mainly include injections, infusions or the like as parenteral administrations. However, the methods for administration are not limited to these examples.

The dose of the cells with induced immunity according to the present invention may generally be from about 10³ to 10¹⁰ cells for a patient once to several times with intervals of a few days and/or a few weeks. However, the dose should be finally determined by clinicians, since it varies depending on the age, body weight and condition of a patient, severity of a disease and the like.

### Examples

The present invention will be explained more specifically by referring to the following examples. However, the scope of the present invention is not limited to the following examples.

### Example 1

Preparation of a complex of cholesterol-modified mannan or pullulan and oncogene product ErbB-2 protein

Hydrophobized polysaccharides were synthesized according to the method of Akiyoshi et al. (Akiyoshi et al., Macromolecules, 26, 3062-3068 (1993)). As the cholesterol-modified mannan, mannan having a molecular weight of about 55,000 (Sigma) modified with about 2.3 cholesterol residues per 100 monosaccharides in average was obtained (abbreviated hereinafter as "CHM-55-2.3"). As the cholesterol-modified pullulan, pullulan having a molecular weight of about 108,000 (Hayashibara Biochemistry Institute) modified with about 0.9 cholesterol residue per 100 monosaccharides in average was obtained (abbreviated hereinafter as "CHP-108-0.9"). In an aqueous solution, each aggregate was found to contain about 7 hydrophobic domains formed by 7 associated cholesterol residues in average.

The above-obtained hydrophobized polysaccharides were dissolved in DMSO, dialyzed against PBS (pH 7.9), and then sonicated by using a probe type sonicator (URP, Tomy Seiko) at 40 W for 10 minutes. The resulting solutions were filtered successively through membranes having a pore size of 1.2 *µ* m, 0.45 *µ* m, and 0.2 *µ* m to obtain aqueous solutions of microparticles comprising the hydrophobized polysaccharides. The concentration of the hydrophobized polysaccharide of each solution was determined as 9.62 mg/ml for CHP-108-0.9, and 6.97 mg/ml for CHM-55-2.3 by the phenol-sulfuric acid method.

A recombinant protein composed of a polypeptide corresponding to amino acid Nos. 1 to 147 of human ErbB-2 protein as an oncogene product (Coussens et al., Science, 230, 1132, 1985), whose N-terminal was fused to a histidine hexamer, was expressed in *Escherichia coli* and used as the antigen protein. A cDNA comprising a region encoding a polypeptide corresponding to the amino acid Nos. 1 to 147 of the human ErbB-2 protein was amplified by PCR using the following two oligonucleotide primers:
TS1: 5'-GGATCCATATGGCTGGCGGCCT-3' (SEQ ID NO:1 in SEQUENCE LISTING), and
TS2: 5'-CGACTGGATCCTATGTGAGACTTC-3' (SEQ ID NO:2 in SEQUENCE LISTING)

The resulting 449 bp DNA fragment was digested with restriction enzymes *Nde*I and *Bam*HI. A plasmid expression vector, pET15b (Novagen), was digested with restriction enzymes *Nde*I and *Bam*HI. The resulting *Nde*I*-Bam*HI fragment of about 5.7 kbp derived from pET15b was ligated to the PCR DNA fragment of the human ErbB-2 cDNA by using a ligation kit (Takara Shuzo), and then used for transformation of *E*. *coli* strain BL21 (DE3) (Novagen). After ampicillin-resistant clones were chosen, a recombinant protein was purified according to the packaged manual by Novagen. Typically, about 20 mg of the recombinant protein was obtained from the recombinant microorganisms cultured in 1 litter of a culture medium.

A solution of the human ErbB-2 recombinant protein (containing 2.0 mg/ml of the protein in PBS/6M urea) was added to the above-obtained aqueous solution of the hydrophobized polysaccharides, and then mixed to obtain a colorless and transparent aqueous solution containing the complex of the hydrophobized polysaccharides and the antigen (5 mg/ml of CHM or CHP, 0.25 mg/ml of the human ErbB-2 recombinant protein). As a result of the DLS (Dynamic Light Scattering) measurement of the solution, it was deduced that complex microparticles having the particle size of about 250 nm and k2/k12 ratio of 0.156 was obtained. The complex of the cholesterol-modified mannan and the ErbB-2 protein thus obtained will be abbreviated hereinafter as "CHM-ErbB2", and the complex of the cholesterol-modified pullulan and the ErbB-2 protein as "CHP-ErbB2".

Under a condition where the same buffer was used but in the absence of the hydrophobized polysaccharides, all the human ErbB-2 recombinant protein was insolubilized and deposited as precipitates.

As a negative control for the immunization experiments, a complex of each hydrophobized polysaccharide and a protein was prepared by using Carbonic Anhydrase II (Sigma, abbreviated hereinafter as "CAB"). The complex of the cholesterol-modified mannan and CAB will be abbreviated hereinafter as "CHM-CAB", and the complex of the cholesterol-modified pullulan and CAB as "CHP-CAB".

### Example 2

Effect of the dendritic cells treated with the complex of the cholesterol-modified pullulan and the oncogene product human ErbB-2 protein on T cell activation (stimulating activity on proliferation of T cells)

A suspension of CHM-ErbB2 prepared in Example 1 was subcutaneously administered for immunization twice with an interval of one week to five female BALB/c mice at the amount of 20 *µ* g per mouse based on protein weight. One week after the final immunization, spleen cells were collected from the immunized cells and T cells were selectively separated with a nylon wool column. The T cells obtained were treated with antibodies and complements using anti-CD4 monoclonal antibody, anti-CD8 monoclonal antibody, and rabbit serum as a complement source to obtain CD4-positive T cells, CD8-positive T cells, and a mixture of CD4-positive and CD8-positive cells.

Separately, marrow cells were collected from six female BALB/c mice and treated with anti-CD4 antibody, anti-CD8 antibody, anti-B cell antibody, and rabbit serum as a complement source to prepare marrow stem cells. The cells obtained were cultured in the presence of GM-CSF (100 units per ml) for six days to prepare dendritic cells derived from the marrow. The dendritic cells obtained were added with a suspension of CHP-ErbB2 or a suspension of CHP-CAB at the protein concentration of 100 *µ* g/ml and co-cultured for three hours. Then, the dendritic cells were washed and further cultured for 18 hours.

The above dendritic cells after the treatment with mitomycin C were subjected to co-cultivation with the CD4-positive T cells, CD8-positive T cells, and cells as a mixture thereof which were obtained from the mice immunized with the CHP-ErbB2 suspension as described above. After 72 hours, ³H-thymidine was added to the culture to observe the proliferation of T cells.

As shown in Fig. 1, each of the CD4-positive T cells, CD8-positive T cells, and the cells as a mixture thereof immunized with the CHP-ErbB2 suspension reacted solely with the dendritic cells treated with CHP-ErbB2 and exhibited cell proliferation, whilst no stimulation on proliferation of the T cells was observed in the dendritic cells treated with the CHP-CAB suspension and the non-treated dendritic cells. In the figure, ErbB2 is represented as HER2.

### Example 3

Effect of the complex of the cholesterol-modified pullulan and the oncogene product human ErbB-2 protein on the ability for presenting antigens of dendritic cells derived from healthy human peripheral blood (cytotoxic activity)

Healthy human peripheral blood monocytes with HLA-A2402 recognizing HER2p63-71 or HLA-A0201 were separated by the Ficoll-Conray method. The cells were cultured on plastic plates for two hours and floating cells were removed. The adherent cells were cultured in the presence of GM-CSF (1,000 units per ml) and IL-4 (100 units per ml) for 10 days. Then, floating cells were collected and used as the dendritic cells derived from marrow.

The resulting dendritic cells were cultured for 3 hours with the CHP-ErbB2 suspension or with the CHP-CAB suspension each at the final protein concentration of 100 *µ* g/ml. The cells were washed twice and further cultured for 16 hours, and then labeled with sodium Cr⁵¹ (50 *µ*l: 100 mCi) to perform a cytotoxic test. For the use as effector cells, healthy human peripheral blood lymphocytes having HLA-A2402 were sensitized with auto-dendritic cells pulsed with the ErbB2p63-71 peptide to induce and establish the CD8-positive HLA-A2402 restrictive CTL clones recognizing the above peptide.

As shown in Fig. 2, the CTL clone was found to have significant cytotoxicity against the HLA-A2402-deriving dendritic cells treated with the CHP-ErbB2 suspension. In contrast, no cytotoxicity was observed against the dendritic cells derived from the same individual, which was treated with the CHP-CAB suspension, and against the dendritic cells from a healthy human having no HLA-A2402 (having HLA-A0201 instead) which was treated with the CHP-ErbB2 suspension or the same cells treated with the CHP-CAB suspension.

### Example 4

Effect of the dendritic cell derived from healthy human peripheral blood which is treated with the complex of the cholesterol-modified pullulan and the oncogene product human ErbB-2 protein on CTL clone activation (stimulating activity on proliferation of CTL clone)

The dendritic cells, prepared from healthy human peripheral blood monocytes having HLA-A2402 as described above, were cultured with the CHP-ErbB2 suspension, the ErbB2 protein alone, the CHP-CAB suspension, or the CAB protein alone each at a protein concentration of 100 *µ* g/ml for 3 hours. The cells were washed and then further cultured for 15 hours.

By using these dendritic cells and the ErbB2 p63-71 peptide-pulsed dendritic cells derived from the same individual as stimulating cells, their abilities of stimulation were examined on the aforementioned CD8-positive HLA-A2402-restrictive CTL clones specific for the ErbB2 p63-71 peptide.

As shown in Fig. 3, the dendritic cells treated with the CHP-ErbB2 suspension and the dendritic cells pulsed with p63-71 peptide deriving from ErbB2 were found to significantly stimulate the CTL clone and induce proliferation compared to the non-treated dendritic cells. These results demonstrate that the CHP-ErbB2 protein acts to have the dendritic cells incorporate the ErbB2 protein and present the p63-71 peptide derived from the protein together with the HLA-A2402 molecules on the dendritic cells.

### Example 5

Anticancer effect of the dendritic cells treated with the complex of the cholesterol-modified pullulan and the oncogene product human ErbB-2 protein

The dendritic cells derived from marrow cells were prepared by the method described in Example 2. The resulting dendritic cells were cultured with the CHP-ErbB2 suspension or the CHP-CAB suspension prepared in Example 1 each at protein concentration of 75 *µ* g/ml for 3 hours. The dendritic cells were washed and further cultured for 18 hours.

To groups of female BALB/c mice each consisting of four mice, recombinant cells CMS7HE were subcutaneously inoculated which were prepared using a fibroblast sarcoma cell strain of the above mouse strain (see, Deleo et al., J. Exp. Med., 146, 720, 1977) so as to express *neo gene* as a selected marker and human ErbB-2 in a conventional manner. After 10 days from the inoculation of the tumor cells, the dendritic cells (4×10⁵ cells) treated by the aforementioned method were subcutaneously administered for immunization with an interval of one week.

The size of the inoculated tumor was measured with time. As shown in Fig. 4, inhibition of the proliferation and complete regression of the tumor were observed in all 4 animals of the 4 mice immunized with the dendritic cells treated with CHP-ErbB2 (DC (CHP-HER2) in Fig.4). In contrast, almost no inhibitory effect on tumor proliferation was observed in the mice immunized with the dendritic cells treated with CHP-CAB or with the non-treated dendritic cells (DC (CHP-CAB) and DC in Fig. 4). These results suggest antigen-specificity and anticancer effect *in vivo* of the above immunization procedure

### Industrial Applicability

In antigen-presenting cells with induced cellular immunity according to the present invention, cellular immunity is induced more efficiently compared to conventional vaccines, and accordingly, the cells can be extremely useful for a cell therapy.

## Claims

1. A cell with induced cellular immunity wherein said cellular immunity is induced by reacting *in vitro* a complex comprising a hydrophobized polysaccharide and an antigen with an antigen-presenting cell.

2. The cell according to claim 1 wherein the antigen-presenting cell is a dendritic cell.

3. The cell according to claim 1 or 2, characterized in that the hydrophobized polysaccharide is a polysaccharide modified with an alkyl group or a sterol residue.

4. The cell according to any one of claims 1 to 3, wherein the hydrophobized polysaccharide is a polysaccharide characterized to contain a saccharide unit, at a ratio of 0.5 to 5 in average per 100 saccharide units that constitute the polysaccharide, whose primary hydroxyl group is a group represented by the formula:
-O-(CH₂)ₘCONH(CH₂)ₙNH-CO-O-R
wherein R represents an alkyl group or a sterol residue; m represents 0 or 1; and n represents a positive integer.

5. The cell according to claim 3 or 4, characterized in that the sterol residue is cholesterol residue.

6. The cell according to any one of claims 1 to 5, characterized in that the polysaccharide is pullulan or mannan.

7. The cell according to any one of claims 1 to 6, characterized in that the antigen is a protein which is presented as an oligopeptide by an MHC class I antigen and induces a cytotoxic T-cell.

8. The cell according to claim 7, wherein the antigen is a tumor cell antigen, a viral antigen, or an autoantigen-reactive T-cell receptor.

9. The cell according to claim 8, characterized in that the antigen is ErbB-2 protein.

10. The cell according to any one of claims 1 to 9, which is characterized to be used as a medicament for parenteral administration.

11. A method for inducing cellular immunity characterized in that said method comprises the step of reacting *in vitro* a complex comprising a hydrophobized polysaccharide and an antigen with an antigen-presenting cell.

12. The method according to claim 11 or 12 characterized in that an amount of the complex comprising a hydrophobized polysaccharide and an antigen is sufficient to induce cellular immunity.

13. A method for inducing cellular immunity in vivo characterized in that the method comprises the steps of isolating an antigen-presenting cell from a living body, reacting a complex comprising a hydrophobized polysaccharide and an antigen with the antigen-presenting cell, and returning the resulting cell to the living body.

14. The method according to claim 13 which comprises the step of returning the antigen-presenting cell to the living body by parenteral administration.
